# EUROPEAN PATENT APPLICATION

(11) **EP 4 070 812 A1**
(43) Date of publication of application: **12.10.2022**
(21) Application number: 22175627.3
(22) Date of filing: 20.01.2017
(51) Int. Cl.: A61K 39/00, A61P 35/00, C07K 16/28

(54) **TREATMENT OF CANCER WITH COMBINATIONS OF IMMUNOREGULATORY AGENTS**

(30) Priority: 21.01.2016 US 201662281571 P; 01.03.2016 US 201662301981 P
(62) Divisional of application: 17742028.8
(71) Applicant: The Board of Trustees of the Leland Stanford Junior University, Stanford, CA 94305-2038 (US)
(72) Inventor: WILLINGHAM, Stephen, Sunnyvale, 94087 (US); HO, Doris Po Yi, Daly City, 94014 (US); MCKENNA, Kelly Marie, Palo Alto, 94306 (US); WEISSMAN, Irving L., Stanford, 94305 (US); VOLKMER, Jens-Peter, Menlo Park, 94025 (US); CHAO, Mark P., Mountain View, 94040 (US); MAJETI, Ravindra, Palo Alto, 94303 (US); MCCRACKEN, Melissa N., Mountain View, 94040 (US)
(74) Representative: Carpmaels & Ransford LLP

(57) **Abstract**

Methods are provided for targeting cells for depletion, including without limitation cancer cells, in a regimen comprising contacting the targeted cells with a combination of immunoregulatory agents. The level of depletion of the targeted cell is enhanced relative to a regimen in which a single agent is used; and the effect may be synergistic relative to a regimen in which a single agent is used.

## Description

### CROSS REFERENCE

This application claims benefit of U.S. Provisional Patent Application No. 62/281,571, filed January 21, 2016 and U.S. Provisional Patent Application No. 62/301,981, filed March 1, 2016, which applications are incorporated herein by reference in their entirety.

### BACKGROUND

The immune system's natural capacity to detect and destroy abnormal cells may prevent the development of many cancers. However, cancer cells are sometimes able to avoid detection and destruction by the immune system. Cancer cells can reduce the expression of tumor antigens on their surface, making it harder for the immune system to detect them; express proteins on their surface that induce immune cell inactivation; and/or induce cells in the microenvironment to release substances that suppress immune responses and promote tumor cell proliferation and survival.

Cancer immunotherapies have been developed to enhance immune responses against tumors, by stimulating specific components of the immune system; or by counteracting signals produced by cancer cells that suppress immune responses.

One approach blocks immune checkpoint proteins, which limit the strength and duration of immune responses. These proteins normally keep immune responses in check by preventing overly intense responses that might damage normal cells as well as abnormal cells. Blocking the activity of immune checkpoint proteins releases the "brakes" on the immune system, increasing its ability to destroy cancer cells.

Immune checkpoint inhibitors in current clinical use include ipilimumab, which blocks the activity of CTLA4, which is expressed on the surface of activated cytotoxic T lymphocytes. CTLA4 acts as a "switch" to inactivate these T cells, thereby reducing the strength of immune responses; inhibiting it increases the cytotoxic T cell response. Two other FDA-approved checkpoint inhibitors, nivolumab and pembrolizumab work in a similar way, but they target PD-1.

Other forms of immunotherapy uses proteins that normally help regulate, or modulate, immune system activity to enhance the body's immune response against cancer, e.g. interleukins and interferons. Antibodies targeted to tumor cell antigens are also in clinical use.

Other forms of immunotherapy exploit the innate immune system. The cell surface protein CD47 on healthy cells and its engagement of a phagocyte receptor, SIRPα, constitutes a key "don't eat-me" signal that can turn off engulfment mediated by multiple modalities, including apoptotic cell clearance and FcR mediated phagocytosis. Blocking the CD47 mediated engagement of SIRPα on a phagocyte, or the loss of CD47 expression in knockout mice, can cause removal of live cells and non-aged erythrocytes. Alternatively, blocking SIRPα recognition also allows engulfment of targets that are not normally phagocytosed. Anti-CD47 antibody treatment has also been shown to not only enable macrophage phagocytosis of cancer, but can also initiate an anti-tumor cytotoxic T cell immune response.

Related publications include "Engineered Sirp alpha Variants As Immunotherapeutic Adjuvants To Anticancer Antibodies." Science 341(6141): 88-91; Willingham, S. B., J. P. Volkmer, Et Al. (2012). "The Cd47-Signal Regulatory Protein Alpha (Sirpa) Interaction Is A Therapeutic Target For Human Solid Tumors." Proc Natl Acad Sci U S A 109(17): 6662-6667. Chao, M. P., A. A. Alizadeh, Et Al. (2010). "Anti-Cd47 Antibody Synergizes With Rituximab To Promote Phagocytosis And Eradicate Non-Hodgkin Lymphoma." Cell 142(5): 699-713.

### SUMMARY

Methods are provided for targeting cells for depletion, including without limitation tumor cells, in a regimen comprising contacting the target and effector cells with a combination of two or more agents that modulate immunoregulatory signaling. In some embodiments the contacting is performed in vivo. In some embodiments the contacting is performed in vitro, e.g. to prime immune effector cells, followed by administration to a subject. In some embodiments, the combination of agents provides a synergistic effect relative to the administration of an agent as a monotherapy. In various embodiments, the combination of immunoregulatory agents is administered in a therapeutic regimen that includes conventional treatment, e.g. targeted anti-tumor antibodies, chemotherapy, radiation therapy, surgery, and the like.

A benefit of the present invention can be the use of lowered doses of the agents relative to the dose required as a single immunoregulatory agent, or a combination of immunoregulatory agents in the absence of CD47 blockade. A benefit of the present invention can also, or alternatively, be a decrease in the length of time required for treatment, relative to the length of time required for treatment as a single immunoregulatory agent, or a combination of immunoregulatory agents in the absence of CD47 blockade. A benefit of the present invention can also, or alternatively, be an enhanced response relative to the response observed after treatment with a single immunoregulatory agent, or a combination of immunoregulatory agents in the absence of CD47 blockade.

Immunoregulatory modulating agents include (i) an agent that blockades CD47 activity; and (ii) one or more of an agent that agonizes an immune costimulatory molecule, e.g. CD40, OX40, etc.; and/or (iii) an agent that antagonizes an immune inhibitory molecule, e.g. CTLA-4, PD1, PDL1, *etc.* The active agents are administered within a period of time to produce an additive or synergistic effect on depletion of cancer cells in the host. Administration may be Methods of administration include, without limitation, systemic administration, intra-tumoral administration, etc. Usually the active agent (i) is administered within about a period of about 45 days, about 30 days, about 21 days, about 14 days, about 10 days, about 8 days, about 7 days, about 6 days, about 5 days, about 4 days, about 3 days, about 2 days, about 1 day or substantially the same day as an agent (ii) and/or (iii). In some embodiments an agent (i) is administered prior to an agent (ii) and/or (iii). In some embodiments an agent (i) is administered after an agent (ii) and/or (iii). The agents can be considered to be combined if administration scheduling is such that the serum level of both agents is at a therapeutic level at the same time. Administration may be repeated as necessary for depletion of the cancer cell population.

In some embodiments, an individual cancer is selected for treatment with a combination therapy of the present invention because the cancer is a cancer type that is responsive to a checkpoint inhibitor, e.g. a PD1 antagonist, a PDL1 antagonist, a CTLA4 antagonist, a TIM-3 antagonist, a BTLA antagonist, a VISTA antagonist, a LAG3 antagonist; etc. In some embodiments, such an immunoregulatory agent is a CTLA-4, PD1 or PDL1 antagonist, e.g. avelumab, nivolumab, pembrolizumab, ipilimumab, and the like. In some such embodiments the cancer is, without limitation, melanoma or small cell lung cancer. In some such embodiments, the cancer is a type that has a high neoantigen, or mutagenesis, burden (see Vogelstein et al. (2013) Science 339(6127):1546-1558, herein specifically incorporated by reference). In other embodiments, the cancer with a type with a low neoantigen burden. In some such embodiments, the combination therapy of the present invention enhances the activity of the checkpoint inhibitor. In other embodiments, where the individual cancer does not respond to a checkpoint inhibitor alone, the combination therapy provides a therapeutic response.

In some embodiments, an individual cancer is selected for treatment with a combination therapy of the present invention because the cancer is a cancer type that is responsive to an immune response agonist, e.g. a CD28 agonist, an OX40 agonist; a GITR agonist, a CD137 agonist, a CD27 agonist, an HVEM agonist, etc. In some embodiments, such an immunoregulatory agent is an OX40, CD137, or GITR agonist e.g. tremelimumab, and the like. In some such embodiments the cancer is, without limitation, melanoma or small cell lung cancer. In some such embodiments, the cancer is a type that has a high neoantigen, or mutagenesis, burden. In other embodiments, the cancer with a type with a low neoantigen burden. In some such embodiments, the combination therapy of the present invention enhances the activity of the agonist. In other embodiments, where the individual cancer does not respond to a agonist alone, the combination therapy provides a therapeutic response.

In some embodiments, an individual cancer is selected for treatment with a combination therapy of the present invention because the cancer is a cancer type that is responsive to a chemokine receptor antagonist, e.g. CCR2, CCR4, etc. In some such embodiments the cancer is, without limitation, a lymphoma, including without limitation T cell lymphoma, e.g. cutaneous T cell lymphoma. In some such embodiments, the cancer is a type that has a high neoantigen, or mutagenesis, burden. In other embodiments, the cancer with a type with a low neoantigen burden. In some such embodiments, the combination therapy of the present invention enhances the activity of the anti-chemokine receptor antagonist. In other embodiments, where the individual cancer does not respond to the antagonist alone, the combination therapy provides a therapeutic response.

The methods of the invention may further comprise administration of an agent that specifically binds to a target cell or an inhibitory immune cell, e.g. an antibody or biologically active fragment or derivative thereof. The level of depletion of the targeted cell, e.g. a cancer cell, an inhibitory immune cell including without limitation regulatory T cells. A number of antibodies are currently in clinical use for the treatment of cancer, and others are in varying stages of clinical development. Antibodies of interest for the methods of the invention may act through ADCC, and may be selective for tumor cells, although one of skill in the art will recognize that some clinically useful antibodies do act on non-tumor cells, e.g. CD20, etc.

In some embodiments a primer agent is administered prior to administering a therapeutically effective dose of an anti-CD47 agent to the individual. Suitable primer agents include an erythropoiesis-stimulating agent (ESA), and/or a priming dose of an anti-CD47 agent. Following administration of the priming agent, and allowing a period of time effective for an increase in reticulocyte production, a therapeutic dose of an anti-CD47 agent is administered. The therapeutic dose can be administered in number of different ways. In some embodiments, two or more therapeutically effective doses are administered after a primer agent is administered. In some embodiments a therapeutically effective dose of an anti-CD47 agent is administered as two or more doses of escalating concentration, in others the doses are equivalent.

In some embodiments, administration of a combination of agents of the invention is combined with an effective dose of an agent that increases patient hematocrit, for example erythropoietin stimulating agents (ESA). Such agents are known and used in the art, including, for example, Aranesp^{®} (darbepoetin alfa), Epogen^{®}NF/Procrit^{®}NF (epoetin alfa), Omontys^{®} (peginesatide), Procrit^{®}, etc.

An anti-CD47 agent for use in the methods of the invention interferes with binding between CD47 present on the cancer cell and SIRPα present on a phagocytic cell. Such methods increase phagocytosis of the cancer cell. Suitable anti-CD47 agents include soluble SIRPα polypeptides; soluble CD47; anti-CD47 antibodies, anti-SIRPa antibodies, and the like, where the term antibodies encompasses antibody fragments and variants thereof, as known in the art. In some embodiments the anti-CD47 agent is an anti-CD47 antibody. In some embodiments the anti-CD47 antibody is a non-hemolytic antibody. In some embodiments the antibody comprises a human IgG4 Fc region.

### BRIEF DESCRIPTION OF THE FIGURES

Figure 1. Effect of combined CD40 agonist and CD47 antagonist.
Figure 2. (A-C) Effect of combined anti-CTLA-4 and CD47 antagonist.

### DETAILED DESCRIPTION OF THE EMBODIMENTS

Methods are provided for the targeted depletion of cancer cells in a subject, where the cancer cells are selectively ablated by immune cell responses to the tumor cells, following contacting with a combination of agents that (i) block CD47 signaling; and (ii) one or more of an agent that agonizes an immune costimulatory molecule, e.g. CD40, OX40, CD28, GITR, CD137, CD27, HVEM, etc.; and/or (iii) an agent that antagonizes an immune inhibitory molecule, e.g. CTLA-4, PD1, PDL1, TIM-3, BTLA, VISTA, LAG-3, *etc.*

To facilitate an understanding of the invention, a number of terms are defined below.

Before the present active agents and methods are described, it is to be understood that this invention is not limited to the particular methodology, products, apparatus and factors described, as such methods, apparatus and formulations may, of course, vary. It is also to be understood that the terminology used herein is for the purpose of describing particular embodiments only, and is not intened to limit the scope of the present invention which will be limited only by appended claims.

It must be noted that as used herein and in the appended claims, the singular forms "a," "and," and "the" include plural referents unless the context clearly dictates otherwise. Thus, for example, reference to "a drug candidate" refers to one or mixtures of such candidates, and reference to "the method" includes reference to equivalent steps and methods known to those skilled in the art, and so forth.

Unless defined otherwise, all technical and scientific terms used herein have the same meaning as commonly understood by one of ordinary skill in the art to which this invention belongs. All publications mentioned herein are incorporated herein by reference for the purpose of describing and disclosing devices, formulations and methodologies which are described in the publication and which might be used in connection with the presently described invention.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Generally, conventional methods of protein synthesis, recombinant cell culture and protein isolation, and recombinant DNA techniques within the skill of the art are employed in the present invention. Such techniques are explained fully in the literature, see, e.g., Maniatis, Fritsch & Sambrook, Molecular Cloning: A Laboratory Manual (1982); Sambrook, Russell and Sambrook, Molecular Cloning: A Laboratory Manual (2001); Harlow, Lane and Harlow, Using Antibodies: A Laboratory Manual: Portable Protocol No. I, Cold Spring Harbor Laboratory (1998); and Harlow and Lane, Antibodies: A Laboratory Manual, Cold Spring Harbor Laboratory; (1988).

### Definitions

*Anti-CD47 agent.* CD47 is a broadly expressed transmembrane glycoprotein with a single Ig-like domain and five membrane spanning regions, which functions as a cellular ligand for SIRPα with binding mediated through the NH2-terminal V-like domain of SIRPα. SIRPα is expressed primarily on myeloid cells, including macrophages, granulocytes, myeloid dendritic cells (DCs), mast cells, and their precursors, including hematopoietic stem cells. Structural determinants on SIRPα that mediate CD47 binding are discussed by Lee et al. (2007) J. Immunol. 179:7741-7750; Hatherley et al. (2008) Mol Cell. 31(2):266-77; Hatherley et al. (2007) J.B.C. 282:14567-75; and the role of SIRPα cis dimerization in CD47 binding is discussed by Lee et al. (2010) J.B.C. 285:37953-63. In keeping with the role of CD47 to inhibit phagocytosis of normal cells, there is evidence that it is transiently upregulated on hematopoietic stem cells (HSCs) and progenitors just prior to and during their migratory phase, and that the level of CD47 on these cells determines the probability that they are engulfed in vivo.

As used herein, the term "anti-CD47 agent" or "agent that provides for CD47 blockade" refers to any agent that reduces the binding of CD47 (e.g., on a target cell) to SIRPα (e.g., on a phagocytic cell). Non-limiting examples of suitable anti-CD47 reagents include SIRPα reagents, including without limitation high affinity SIRPα polypeptides, anti-SIRPa antibodies, soluble CD47 polypeptides, and anti-CD47 antibodies or antibody fragments. In some embodiments, a suitable anti-CD47 agent (e.g. an anti-CD47 antibody, a SIRPα reagent, etc.) specifically binds CD47 to reduce the binding of CD47 to SIRPα.

In some embodiments, a suitable anti-CD47 agent (e.g., an anti-SIRPa antibody, a soluble CD47 polypeptide, etc.) specifically binds SIRPα to reduce the binding of CD47 to SIRPα. A suitable anti-CD47 agent that binds SIRPα does not activate SIRPα (e.g., in the SIRPα-expressing phagocytic cell). The efficacy of a suitable anti-CD47 agent can be assessed by assaying the agent. In an exemplary assay, target cells are incubated in the presence or absence of the candidate agent and in the presence of an effector cell, e.g. a macrophage or other phagocytic cell. An agent for use in the methods of the invention will up-regulate phagocytosis by at least 5% (e.g., at least 10%, at least 20%, at least 30%, at least 40%, at least 50%, at least 60%, at least 70%, at least 80%, at least 90%, at least 100%, at least 120%, at least 140%, at least 160%, at least 180%, at least 200%, at least 500%, at least 1000%) compared to phagocytosis in the absence of the agent. Similarly, an *in vitro* assay for levels of tyrosine phosphorylation of SIRPα will show a decrease in phosphorylation by at least 5% (e.g., at least 10%, at least 15%, at least 20%, at least 30%, at least 40%, at least 50%, at least 60%, at least 70%, at least 80%, at least 90%, or 100%) compared to phosphorylation observed in absence of the candidate agent.

In some embodiments, the anti-CD47 agent does not activate CD47 upon binding. When CD47 is activated, a process akin to apoptosis (i.e., programmed cell death) may occur (Manna and Frazier, Cancer Research, 64, 1026-1036, Feb. 1 2004). Thus, in some embodiments, the anti-CD47 agent does not directly induce cell death of a CD47-expressing cell.

In some embodiments a primer agent is administered prior to administering a therapeutically effective dose of an anti-CD47 agent to the individual. Suitable primer agents include an erythropoiesis-stimulating agent (ESA), and/or a priming dose of an anti-CD47 agent. Following administration of the priming agent, and allowing a period of time effective for an increase in reticulocyte production, a therapeutic dose of an anti-CD47 agent is administered. Administration may be made in accordance with the methods described in copending patent application USSN 14/769,069, herein specifically incorporated by reference.

SIRPα *reagent.* A SIRPα reagent comprises the portion of SIRPα that is sufficient to bind CD47 at a recognizable affinity, which normally lies between the signal sequence and the transmembrane domain, or a fragment thereof that retains the binding activity. A suitable SIRPα reagent reduces (e.g., blocks, prevents, etc.) the interaction between the native proteins SIRPα and CD47. The SIRPα reagent will usually comprise at least the d1 domain of SIRPα.

In some embodiments, a subject anti-CD47 agent is a "high affinity SIRPα reagent", which includes SIRPα -derived polypeptides and analogs thereof (e.g., CV1-hlgG4, and CV1 monomer). High affinity SIRPα reagents are described in international application PCT/US13/21937, which is hereby specifically incorporated by reference. High affinity SIRPα reagents are variants of the native SIRPα protein. The amino acid changes that provide for increased affinity are localized in the d1 domain, and thus high affinity SIRPα reagents comprise a d1 domain of human SIRPα, with at least one amino acid change relative to the wild-type sequence within the d1 domain. Such a high affinity SIRPα reagent optionally comprises additional amino acid sequences, for example antibody Fc sequences; portions of the wild-type human SIRPα protein other than the d1 domain, including without limitation residues 150 to 374 of the native protein or fragments thereof, usually fragments contiguous with the d1 domain; and the like. High affinity SIRPα reagents may be monomeric or multimeric, i.e. dimer, trimer, tetramer, *etc.* In some embodiments, a high affinity SIRPα reagent is soluble, where the polypeptide lacks the SIRPα transmembrane domain and comprises at least one amino acid change relative to the wild-type SIRPα sequence, and wherein the amino acid change increases the affinity of the SIRPα polypeptide binding to CD47, for example by decreasing the off-rate by at least 10-fold, at least 20-fold, at least 50-fold, at least 100-fold, at least 500-fold, or more.

Optionally the SIRPα reagent is a fusion protein, e.g., fused in frame with a second polypeptide. In some embodiments, the second polypeptide is capable of increasing the size of the fusion protein, e.g., so that the fusion protein will not be cleared from the circulation rapidly. In some embodiments, the second polypeptide is part or whole of an immunoglobulin Fc region. The Fc region aids in phagocytosis by providing an "eat me" signal, which enhances the block of the "don't eat me" signal provided by the high affinity SIRPα reagent. In other embodiments, the second polypeptide is any suitable polypeptide that is substantially similar to Fc, e.g., providing increased size, multimerization domains, and/or additional binding or interaction with Ig molecules.

*Anti-CD47 antibodies.* In some embodiments, a subject anti-CD47 agent is an antibody that specifically binds CD47 (i.e., an anti-CD47 antibody) and reduces the interaction between CD47 on one cell (e.g., an infected cell) and SIRPα on another cell (e.g., a phagocytic cell). In some embodiments, a suitable anti-CD47 antibody does not activate CD47 upon binding. Some anti-CD47 antibodies do not reduce the binding of CD47 to SIRPα (and are therefore not considered to be an "anti-CD47 agent" herein) and such an antibody can be referred to as a "non-blocking anti-CD47 antibody." A suitable anti-CD47 antibody that is an "anti-CD47 agent" can be referred to as a "CD47-blocking antibody". Non-limiting examples of suitable antibodies include clones B6H12, 5F9, 8B6, and C3 (for example as described in International Patent Publication WO 2011/143624, herein specifically incorporated by reference). Suitable anti-CD47 antibodies include fully human, humanized or chimeric versions of such antibodies. Humanized antibodies (e.g., hu5F9-G4) are especially useful for in vivo applications in humans due to their low antigenicity. Similarly caninized, felinized, etc. antibodies are especially useful for applications in dogs, cats, and other species respectively. Antibodies of interest include humanized antibodies, or caninized, felinized, equinized, bovinized, porcinized, etc., antibodies, and variants thereof.

In some embodiments an anti-CD47 antibody comprises a human IgG Fc region, e.g. an IgG1, IgG2a, IgG2b, IgG3, IgG4 constant region. In a preferred embodiment the IgG Fc region is an IgG4 constant region. The IgG4 hinge may be stabilized by the amino acid substitution S241P (see Angal et al. (1993) Mol. Immunol. 30(1):105-108, herein specifically incorporated by reference).

*Anti-SIRPa antibodies.* In some embodiments, a subject anti-CD47 agent is an antibody that specifically binds SIRPα (i.e., an anti-SIRPa antibody) and reduces the interaction between CD47 on one cell (e.g., an infected cell) and SIRPα on another cell (e.g., a phagocytic cell). Suitable anti-SIRPa antibodies can bind SIRPα without activating or stimulating signaling through SIRPα because activation of SIRPα would inhibit phagocytosis. Instead, suitable anti-SIRPα antibodies facilitate the preferential phagocytosis of inflicted cells over normal cells. Those cells that express higher levels of CD47 (e.g., infected cells) relative to other cells (non-infected cells) will be preferentially phagocytosed. Thus, a suitable anti-SIRPa antibody specifically binds SIRPα (without activating/stimulating enough of a signaling response to inhibit phagocytosis) and blocks an interaction between SIRPα and CD47. Suitable anti-SIRPa antibodies include fully human, humanized or chimeric versions of such antibodies. Humanized antibodies are especially useful for in vivo applications in humans due to their low antigenicity. Similarly caninized, felinized, etc. antibodies are especially useful for applications in dogs, cats, and other species respectively. Antibodies of interest include humanized antibodies, or caninized, felinized, equinized, bovinized, porcinized, etc., antibodies, and variants thereof.

*Soluble CD47 polypeptides.* In some embodiments, a subject anti-CD47 agent is a soluble CD47 polypeptide that specifically binds SIRPα and reduces the interaction between CD47 on one cell (e.g., an infected cell) and SIRPα on another cell (e.g., a phagocytic cell). A suitable soluble CD47 polypeptide can bind SIRPα without activating or stimulating signaling through SIRPα because activation of SIRPα would inhibit phagocytosis. Instead, suitable soluble CD47 polypeptides facilitate the preferential phagocytosis of infected cells over non-infected cells. Those cells that express higher levels of CD47 (e.g., infected cells) relative to normal, non-target cells (normal cells) will be preferentially phagocytosed. Thus, a suitable soluble CD47 polypeptide specifically binds SIRPα without activating/stimulating enough of a signaling response to inhibit phagocytosis.

In some cases, a suitable soluble CD47 polypeptide can be a fusion protein (for example as structurally described in US Patent Publication US20100239579, herein specifically incorporated by reference). However, only fusion proteins that do not activate/stimulate SIRPα are suitable for the methods provided herein. Suitable soluble CD47 polypeptides also include any peptide or peptide fragment comprising variant or naturally existing CD47 sequences (e.g., extracellular domain sequences or extracellular domain variants) that can specifically bind SIRPα and inhibit the interaction between CD47 and SIRPα without stimulating enough SIRPα activity to inhibit phagocytosis.

In certain embodiments, soluble CD47 polypeptide comprises the extracellular domain of CD47, including the signal peptide, such that the extracellular portion of CD47 is typically 142 amino acids in length. The soluble CD47 polypeptides described herein also include CD47 extracellular domain variants that comprise an amino acid sequence at least 65%-75%, 75%-80%, 80-85%, 85%-90%, or 95%-99% (or any percent identity not specifically enumerated between 65% to 100%), which variants retain the capability to bind to SIRPα without stimulating SIRPα signaling.

In certain embodiments, the signal peptide amino acid sequence may be substituted with a signal peptide amino acid sequence that is derived from another polypeptide (e.g., for example, an immunoglobulin or CTLA4). For example, unlike full-length CD47, which is a cell surface polypeptide that traverses the outer cell membrane, the soluble CD47 polypeptides are secreted; accordingly, a polynucleotide encoding a soluble CD47 polypeptide may include a nucleotide sequence encoding a signal peptide that is associated with a polypeptide that is normally secreted from a cell.

In other embodiments, the soluble CD47 polypeptide comprises an extracellular domain of CD47 that lacks the signal peptide. As described herein, signal peptides are not exposed on the cell surface of a secreted or transmembrane protein because either the signal peptide is cleaved during translocation of the protein or the signal peptide remains anchored in the outer cell membrane (such a peptide is also called a signal anchor). The signal peptide sequence of CD47 is believed to be cleaved from the precursor CD47 polypeptide in vivo.

In other embodiments, a soluble CD47 polypeptide comprises a CD47 extracellular domain variant. Such a soluble CD47 polypeptide retains the capability to bind to SIRPα without stimulating SIRPα signaling. The CD47 extracellular domain variant may have an amino acid sequence that is at least 65%-75%, 75%-80%, 80-85%, 85%-90%, or 95%-99% identical (which includes any percent identity between any one of the described ranges) to the native CD47 sequence.

*Immune Responsiveness Modulators.* Immune checkpoint proteins are immune inhibitory molecules that act to decrease immune responsiveness toward a target cell, particularly against a tumor cell in the methods of the invention. Endogenous responses to tumors by T cells can be dysregulated by tumor cells activating immune checkpoints (immune inhibitory proteins) and inhibiting co-stimulatory receptors (immune activating proteins). The class of therapeutic agents referred to in the art as "immune checkpoint inhibitors" reverses the inhibition of immune responses through administering antagonists of inhibitory signals. Other immunotherapies administer agonists of immune costimulatory molecules to increase responsiveness. In some embodiments, an *in vitro* assay of T cell activation, including without limitation assays in the Examples, is used in the determination of specific combinations and dosing schedules.

The immune-checkpoint receptors that have been most actively studied in the context of clinical cancer immunotherapy, cytotoxic T-lymphocyte-associated antigen 4 (CTLA4; also known as CD152) and programmed cell death protein 1 (PD1; also known as CD279) -are both inhibitory receptors. The clinical activity of antibodies that block either of these receptors implies that antitumor immunity can be enhanced at multiple levels and that combinatorial strategies can be intelligently designed, guided by mechanistic considerations and preclinical models.

CTLA4 is expressed exclusively on T cells where it primarily regulates the amplitude of the early stages of T cell activation. CTLA4 counteracts the activity of the T cell co-stimulatory receptor, CD28. CD28 and CTLA4 share identical ligands: CD80 (also known as B7.1) and CD86 (also known as B7.2). The major physiological roles of CTLA4 are downmodulation of helper T cell activity and enhancement of regulatory T (TReg) cell immunosuppressive activity. CTLA4 blockade results in a broad enhancement of immune responses. Two fully humanized CTLA4 antibodies, ipilimumab and tremelimumab, are in clinical testing and use. Clinically the response to immune-checkpoint blockers is slow and, in many patients, delayed up to 6 months after treatment initiation. In some cases, metastatic lesions actually increase in size on computed tomography (CT) or magnetic resonance imaging (MRI) scans before regressing.

Anti-CTLA4 antibodies that antagonize this inhibitory immune function are very potent therapeutics but have significant side effects since this enables also T cell activity against the self that is usually inhibited through these inhibitory molecules and pathways. A combination with an agent that blockades CD47 activity is beneficial to minimize these side effects for the following reasons. The anti-CD47 agent may be given before the therapy with the anti-CTLA4 agent and stimulate a specific anti-tumor T cell response (*Anti-CD47 antibody-mediated phagocytosis of cancer by macrophages primes an effective antitumor T-cell response;* Tseng et al., Proc Natl Acad Sci U S A. 2013 Jul 2;110(27):11103-8*.).* A subsequent anti-CTLA4 therapy will allow the activity and expansion of the specific anti-tumor T cells. But a shorter treatment period and/or lower dose can be used relative to anti-CTLA4 monotherapy, since the specific and potent anti-tumor T cells have been induced prior to treatment with anti-CTLA4 and thus the therapeutic effect of the anti-CTLA4 therapy can be achieved with less side effects. Treatment with anti-CTLA4 may be (a) after anti-CD47 agent, potentially with a lower dose or (b) simultaneously with anti-CD47 agent but at a lower dose. Alternatively the anti-CTLA4 dose and duration of treatment may be at conventional levels, with enhanced potency when combined with anti-CD47.

In some embodiments the dose of anti-CTLA4 agent is reduced to a level that minimizes undesirable side effects, e.g. at a dose that is up to about 90% of the currently approvdes dose, that is up to about 80%, up to about 70%, up to about 60%, up to about 50%, up to about 40%, up to about 30%, up to about 20%, up to about 10%, up to about 5% of a conventional dose. In some embodiments the number of doses is reduced, e.g. dosing with anti-CTLA4 agent not more than 1X, not more than 2X, not more than 3X, etc. As a reference, for example, current protocols usually call for administration of ipilimumab at a dose of 3 mg/kg, administered every 3 weeks for a total of 4 doses; optionally in combination with additional agents such as, for example dacarbazine or temozolomide; or with peptide vaccines. Other protocols have explored administration of ipilimumab at the dose of 10 mg/kg as a single agent against metastatic melanoma.

Tremelimumab has been administered as a single antibody infusion at doses ranging from 0.01 mg/kg to 15 mg/kg. Objective responses were evident at doses of 3 mg/kg and above. The majority of responses were noted in patients that achieved sustained plasma levels of tremelimumab beyond 30 µg/ml at one month. The doses of 10 mg/kg administered every month and 15 mg/kg administered every 3 months have been studied further in a phase II randomized clinical trial, however toxicity was doubled when dosing more frequently with the 10 mg/kg monthly regimen. Based on these data, single agent tremelimumab at 15 mg/kg every 3 months was chosen for clinical trials.

For some patients, the ability of CTLA-4 blockade to activate the immune system results in inflammatory manifestations characterized as immune-related adverse events (irAEs). The most clinically significant irAE is enterocolitis which can range in severity; grade III/IV enterocolitis is seen in ~15% of patients treated with ipilimumab at 10 mg/kg. Additional irAEs include rash/pruritus (>50%), hepatitis (5-10%), hypophysitis (5%), uveitis (<2%), pancreatitis (<2%), and leucopenia (<2%). The combination with an anti-CD47 agent may reduce such adverse events. In some embodiments of the invention a method is provided for treating cancer with a combination of an agent that inhibits CTLA-4 and an anti-CD47 agent, where the dosing of agents in the combination provides for a treatment of cancer with a clinically significant reduction in immune-related adverse events relative to the dosing required for anti-CTLA-4 in the absence of an anti-CD47 agent.

CTLA4 is expressed on regulatory T cells that inhibit T cell activation and expansion and anti-CTLA4 antibodies block their inhibitory immunosuppressive function. As a result, anti-tumor T cells can be/stay activated and expand. One aspect of this effect is the inhibition of the inhibitory signaling pathway but another aspect is the depletion of regulatory T cells that express CTLA4. Thus, in order to get a very potent anti-tumor effect it might be desired to enhance the depletion of the regulatory T cells. The depletion is mediated through ADCP, ADCC, and/or CDC. Anti-CD47 agents can synergize with targeted monoclonal antibodies and enhance their potency to stimulate ADCP and ADCC (Anti-CD47 antibody synergizes with rituximab to promote phagocytosis and eradicate non-Hodgkin lymphoma, Chao et al., Cell. 2010 Sep 3;142(5):699-713.) Thus a combination of anti-CTLA4 agents with anti-CD47 agents could enhance the potency. Anti-CD47 can be given with anti-CTLA4. Since the potency would be enhanced the duration of anti-CTLA4 therapy can be reduced compared to current treatment regimens.

Other immune-checkpoint proteins are PD1 and PDL1. Antibodies in current clinical use against these targets include nivolumab and pembrolizumab. The major role of PD1 is to limit the activity of T cells in peripheral tissues at the time of an inflammatory response to infection and to limit autoimmunity. PD1 expression is induced when T cells become activated. When engaged by one of its ligands, PD1 inhibits kinases that are involved in T cell activation. PD1 is highly expressed on T_{Reg} cells, where it may enhance their proliferation in the presence of ligand. Because many tumors are highly infiltrated with T_{Reg} cells, blockade of the PD1 pathway may also enhance antitumor immune responses by diminishing the number and/or suppressive activity of intratumoral T_{Reg} cells.

The two ligands for PD1 are PD1 ligand 1 (PDL1; also known as B7-H1 and CD274) and PDL2 (also known as B7-DC and CD273). The PD1 ligands are commonly upregulated on the tumor cell surface from many different human tumors. On cells from solid tumors, the major PD1 ligand that is expressed is PDL1. PDL1 is expressed on cancer cells and through binding to it's receptor PD1 on T cells it inhibits T cell activation/function. Therefore, PD1 and PDL1 blocking agents can overcome this inhibitory signaling and maintain or restore anti-tumor T cell function. Anti-CD47 agents can stimulate a specific anti-tumor T cell response (*Anti-CD47 antibody-mediated phagocytosis of cancer by macrophages primes an effective antitumor T-cell response;* Tseng et al., Proc Natl Acad Sci U S A. 2013 Jul 2;110(27):11103-8*.)* Anti-PD1/PDL1 agents can enhance the efficacy of anti-CD47 agents. The CD47 agent may be administered in combination or prior to the anti-PD1/PDL1 agents to simulate priming of tumor-specific T cells that can expand if the inhibitory anti-PD1/PDL1 pathway is blocked.

PDL1 is expressed on cancer cells and through binding to its receptor PD1 on T cells it inhibits T cell activation/function. Therefore, PD1 and PDL1 blocking agents can overcome this inhibitory signaling and maintain or restore anti-tumor T cell function. However, since PDL1 is expressed on tumor cells, antibodies that bind and block PDL1 can also enable ADCP, ADCC, and CDC of tumor cells. Anti-CD47 agents can synergize with targeted monoclonal antibodies and enhance their potency to stimulate ADCP and ADCC (Anti-CD47 antibody synergizes with rituximab to promote phagocytosis and eradicate non-Hodgkin lymphoma, Chao et al., Cell. 2010 Sep 3;142(5):699-713.) Thus a combination of anti-PDL1 agents with anti-CD47 agents can enhance the anti-tumor potency. These agents may be administered together (over the same course of treatment, not necessarily the same day and frequency).

Lymphocyte activation gene 3 (LAG3; also known as CD223), 2B4 (also known as CD244), B and T lymphocyte attenuator (BTLA; also known as CD272), T cell membrane protein 3 (TIM3; also known as HAVcr2), adenosine A2a receptor (A2aR) and the family of killer inhibitory receptors have each been associated with the inhibition of lymphocyte activity and in some cases the induction of lymphocyte anergy. Antibody targeting of these receptors can be used in the methods of the invention.

LAG3 is a CD4 homolog that enhances the function of T_{Reg} cells. LAG3 also inhibits CD8⁺ effector T cell functions independently of its role on T_{Reg} cells. The only known ligand for LAG3 is MHC class II molecules, which are expressed on tumor-infiltrating macrophages and dendritic cells. LAG3 is one of various immune-checkpoint receptors that are coordinately upregulated on both T_{Reg} cells and anergic T cells, and simultaneous blockade of these receptors can result in enhanced reversal of this anergic state relative to blockade of one receptor alone. In particular, PD1 and LAG3 are commonly co-expressed on anergic or exhausted T cells. Dual blockade of LAG3 and PD1 synergistically reversed anergy among tumor-specific CD8⁺ T cells and virus-specific CD8⁺ T cells in the setting of chronic infection. LAG3 blocking agents can overcome this inhibitory signaling and maintain or restore anti-tumor T cell function. Anti-CD47 agents can stimulate a specific anti-tumor T cell response (*Anti-CD47 antibody-mediated phagocytosis of cancer by macrophages primes an effective antitumor T-cell response;* Tseng et al., Proc Natl Acad Sci U S A. 2013 Jul 2;110(27):11103-8*.)* Therefore, LAG3 agents can enhance the efficacy of anti-CD47 agents. The CD47 agent may be given in combination or prior to the anti-LAG3 agent to stimulate priming of tumor-specific T cells that can expand if the inhibitory anti-LAG3 pathway is blocked.

TIM3 inhibits T helper 1 (T_{H}1) cell responses, and TIM3 antibodies enhance antitumor immunity. TIM3 has also been reported to be co-expressed with PD1 on tumor-specific CD8⁺ T cells. Tim3 blocking agents can overcome this inhibitory signaling and maintain or restore anti-tumor T cell function. Anti-CD47 agents can stimulate a specific anti-tumor T cell response *(Anti-CD47 antibody-mediated phagocytosis of cancer by macrophages primes an effective antitumor T-cell response;* Tseng et al., Proc Natl Acad Sci USA. 2013 Jul 2;110(27):11103-8*.)* Therefore, Tim3 agents can enhance the efficacy of anti-CD47 agents. The CD47 agent may be given in combination or prior to the anti-Tim3 agent to stimulate priming of tumor-specific T cells that can expand if the inhibitory anti-Tim3 pathway is blocked.

BTLA is an inhibitory receptor on T cells that interacts with TNFRSF14. BTLA^{hi} T cells are inhibited in the presence of its ligand. The system of interacting molecules is complex: CD160 (an immunoglobulin superfamily member) and LIGHT (also known as TNFSF14), mediate inhibitory and co-stimulatory activity, respectively. Signaling can be bidirectional, depending on the specific combination of interactions. Dual blockade of BTLA and PD1 enhances antitumor immunity.

A2aR, the ligand of which is adenosine, inhibits T cell responses, in part by driving CD4⁺ T cells to express FOXP3 and hence to develop into T_{Reg} cells. Deletion of this receptor results in enhanced and sometimes pathological inflammatory responses to infection. A2aR can be inhibited either by antibodies that block adenosine binding or by adenosine analogues.

Agents that agonize an immune costimulatory molecule are also useful in the methods of the invention. Such agents include agonists or CD40 and OX40. CD40 is a costimulatory protein found on antigen presenting cells (APCs) and is required for their activation. These APCs include phagocytes (macrophages and dendritic cells) and B cells. CD40 is part of the TNF receptor family. The primary activating signaling molecules for CD40 are IFNγ and CD40 ligand (CD40L). Stimulation through CD40 activates macrophages. One of the major effects of CD47 blocking agents is to enhance phagocytosis of target cells by macrophages and other phagocytes. Therefore, combining agonistic CD40 ligands with anti CD47 can enhance the therapeutic efficacy compared to each mono therapy (example 1). Agonistic CD40 agents may be administered substantially simultaneously with anti-CD47 agents; or may be administered prior to and concurrently with treatment with anti-CD47 to pre-activate macrophages.

OX40 (CD134) is a member of the TNFR super-family and expressed on T cells. Molecules that bind OX40 can stimulate proliferation and differentiation of T cells. Anti-CD47 agents can stimulate a specific anti-tumor T cell response (*Anti-CD47 antibody-mediated phagocytosis of cancer by macrophages primes an effective antitumor T-cell response;* Tseng et al., Proc Natl Acad Sci U S A. 2013 Jul 2;110(27):11103-8*.)* Agonistic OX40 agents can enhance the efficacy of anti-CD47 agents. Agonistic OX40 agents may be administered substantially simultaneously with anti-CD47 agents; or may be administered prior to and concurrently with treatment with anti-CD47 to simulate priming of tumor-specific T cell clones that can be expanded through the OX40 agent.

Other immuno-oncology agents that can be administered in combination with CD47 blockade according to the methods described herein include antibodies specific for chemokine receptors, including without limitation anti-CCR4 and anti-CCR2. Anti CCR4 (CD194) antibodies of interest include humanized monoclonal antibodies directed against C-C chemokine receptor 4 (CCR4) with potential anti-inflammatory and antineoplastic activities. Exemplary is mogamulizumab, which selectively binds to and blocks the activity of CCR4, which may inhibit CCR4-mediated signal transduction pathways and, so, chemokine-mediated cellular migration and proliferation of T cells, and chemokine-mediated angiogenesis. In addition, this agent may induce antibody-dependent cell-mediated cytotoxicity (ADCC) against CCR4-positive T cells. CCR4, a G-coupled-protein receptor for C-C chemokines such MIP-1, RANTES, TARC and MCP-1, is expressed on the surfaces of some types of T cells, endothelial cells, and some types of neurons. CCR4, also known as CD194, may be overexpressed on adult T-cell lymphoma (ATL) and peripheral T-cell lymphoma (PTCL) cells.

Anti-CCR4 Ab may be administered in combination with an agent for CD47 blockade for enhanced depletion of CCR4 positive target cells, including without limitation T-cell lymphoma, especially cutaneous T cell lymphoma (CTCL), or DLBCL, breast cancer, renal cell carcinoma, colon cancer, other. CD47 blockade can synergize with cancer targeting monoclonal antibodies and enhance their efficacy for ADCP and ADCC. In some such embodiments,CD47 blockade and anti-CCR4 can be administered at substantially the same time.

Anti-CCR4 Ab may be administered in combination with an agent for CD47 blockade for enhanced depletion of CCR4 positive regulatory T cells. CCR4 is expressed on regulatory T cells and in involved in mediating recruitment of regulatory T cells into tumors. Regulatory T cells suppress a response for anti-tumor T cells and thus their inhibition or depletion is desired. CD47 blockade can synergize with targeted monoclonal antibodies and enhance their efficacy for ADCP and ADCC and thus enhance depletion of regulatory T cells. In some such embodiments,CD47 blockade and anti-CCR4 can be administered at substantially the same time.

Macrophages and other antigen presenting cells can prime an anti-tumor T cells response upon anti-CD47 Ab mediated phagocytosis through antigen cross presentation. In related embodiments, to protect these anti-tumor T cells from inhibition or depletion through regulatory T cells CD47 blockade is combined with anti-CCR4 Ab therapy. In such embodiments, the anti-CD47 agent and the anti-CCR4 Ab may be administered at the same time; the anti-CD47 agent may be administered prior to the anti-CCR4 antibody to prime a T cells response before administering anti-CCR4 Ab to inhibit or deplete regulatory T cells; the anti-CD47 agent may be given after the anti-CCR4 antibody to inhibit or deplete regulatory T cells before priming a T cells response against the tumor.

The combination therapy described above may be combined with other agents that act on regulatory T cells, e.g. anti-CTLA4 Ab, or other T cell checkpoint inhibitors, e.g. anti-PD1, anti-PDL1 antibodies, and the like.

Anti-CCR2 (CD192) Ab. CCR2 is expressed on inflammatory macrophages that can be found in various inflammatory conditions, e.g. rheumatoid arthritis; and have also been identified as expressed on tumor promoting macrophages. Chemokines that bind to CCR2, e.g. CCL2, can recruit and activate the inflammatory macrophages. Inhibiting the chemokine signaling through CCR2 with anti-CCR2 antibodies may result in lower frequencies of undesirable autoimmune or tumor promoting macrophages through inhibition of recruiting or antibody dependent depletion, resulting in mitigation of autoimmune diseases like rheumatoid arthritis, or inhibition of tumor growth or metastasis. CCR2 is also expressed on regulatory T cells, and the CCR2 ligand, CCL2, mediates recruitment of regulatory T cells into tumors. Regulatory T cells suppress a response for anti-tumor T cells and thus their inhibition or depletion is desired.

Anti-CCR2 Ab is administered in combination with CD47 blockade for enhanced depletion of CCR2 positive target cells. These target cells can be human multiple myeloma, prostate cancer, and the like. CD47 blockade can synergize with cancer targeting monoclonal antibodies and enhance their efficacy for ADCP and ADCC. In some such embodiments,CD47 blockade and anti-CCR2 can be administered at substantially the same time.

Anti-CCR2 Ab is administered in combination with CD47 blockade for enhanced depletion of CCR2 positive inflammatory and tumor promoting macrophages and regulatory T cells. CCR2 is expressed on inflammatory and tumor promoting macrophages and regulatory T cells and CCL2 a ligand to CCR2 mediates recruitment of inflammatory and tumor promoting macrophages and regulatory T cells into tumors. Inflammatory (tumor associated macrophages) and regulatory T cells suppress an anti-tumor immune response and therefore their inhibition or depletion is desired. CD47 Ab can synergize with targeted monoclonal antibodies and enhance their efficacy for ADCP and ADCC and thus enhance depletion of inflammatory (tumor associated macrophages) and regulatory T cells. In some such embodiments,CD47 blockade and anti-CCR2 can be administered at substantially the same time.

Anti-CCR2 Ab is administered in combination with CD47 blockade for enhanced anti-tumor activity. Macrophages and other antigen presenting cells can prime an anti-tumor T cells response upon anti-CD47 mediated phagocytosis through antigen cross presentation. To protect these anti-tumor T cells from inhibition or depletion through inflammatory and tumor promoting macrophages and regulatory T cells the anti-CD47 blockade is combined with anti-CCR2 Ab therapy. In such embodiments, the anti-CD47 agent and the anti-CCR2 Ab may be administered at the same time; the anti-CD47 agent may be administered prior to the anti-CCR2 antibody; the anti-CD47 agent may be given after the anti-CCR2.

The combination therapy described above may be combined with other agents that act on regulatory T cells, e.g. anti-CTLA4 Ab, or other T cell checkpoint inhibitors, e.g. anti-PD1, anti-PDL1 antibodies, and the like.

As used herein, "antibody" includes reference to an immunoglobulin molecule immunologically reactive with a particular antigen, and includes both polyclonal and monoclonal antibodies. The term also includes genetically engineered forms such as chimeric antibodies (e.g., humanized murine antibodies) and heteroconjugate antibodies. The term "antibody" also includes antigen binding forms of antibodies, including fragments with antigen-binding capability (*e.g*., Fab', F(ab')₂, Fab, Fv and rlgG. The term also refers to recombinant single chain Fv fragments (scFv). The term antibody also includes bivalent or bispecific molecules, diabodies, triabodies, and tetrabodies.

Selection of antibodies may be based on a variety of criteria, including selectivity, affinity, cytotoxicity, *etc.* The phrase "specifically (or selectively) binds" to an antibody or "specifically (or selectively) immunoreactive with," when referring to a protein or peptide, refers to a binding reaction that is determinative of the presence of the protein, in a heterogeneous population of proteins and other biologics. Thus, under designated immunoassay conditions, the specified antibodies bind to a particular protein sequences at least two times the background and more typically more than 10 to 100 times background. In general, antibodies of the present invention bind antigens on the surface of target cells in the presence of effector cells (such as natural killer cells or macrophages). Fc receptors on effector cells recognize bound antibodies.

An antibody immunologically reactive with a particular antigen can be generated by recombinant methods such as selection of libraries of recombinant antibodies in phage or similar vectors, or by immunizing an animal with the antigen or with DNA encoding the antigen. Methods of preparing polyclonal antibodies are known to the skilled artisan. The antibodies may, alternatively, be monoclonal antibodies. Monoclonal antibodies may be prepared using hybridoma methods. In a hybridoma method, an appropriate host animal is typically immunized with an immunizing agent to elicit lymphocytes that produce or are capable of producing antibodies that will specifically bind to the immunizing agent. Alternatively, the lymphocytes may be immunized *in vitro.* The lymphocytes are then fused with an immortalized cell line using a suitable fusing agent, such as polyethylene glycol, to form a hybridoma cell.

Human antibodies can be produced using various techniques known in the art, including phage display libraries. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire.

Antibodies also exist as a number of well-characterized fragments produced by digestion with various peptidases. Thus pepsin digests an antibody below the disulfide linkages in the hinge region to produce F(ab)'₂, a dimer of Fab which itself is a light chain joined to V_{H}-C_{H1} by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region. While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries.

A "humanized antibody" is an immunoglobulin molecule which contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin.

Antibodies of interest may be tested for their ability to induce ADCC (antibody-dependent cellular cytotoxicity) or ADCP (antibody dependent cellular phagocytosis). Antibody-associated ADCC activity can be monitored and quantified through detection of either the release of label or lactate dehydrogenase from the lysed cells, or detection of reduced target cell viability (e.g. annexin assay). Assays for apoptosis may be performed by terminal deoxynucleotidyl transferase-mediated digoxigenin-11-dUTP nick end labeling (TUNEL) assay (Lazebnik et al., Nature: 371, 346 (1994). Cytotoxicity may also be detected directly by detection kits known in the art, such as Cytotoxicity Detection Kit from Roche Applied Science (Indianapolis, Ind.).

A number of antibodies that target tumor cell antigens are currently in clinical use for the treatment of cancer, and others are in varying stages of clinical development. For example, there are a number of antigens and corresponding monoclonal antibodies for the treatment of B cell malignancies. One target antigen is CD20. Rituximab is a chimeric unconjugated monoclonal antibody directed at the CD20 antigen. CD20 has an important functional role in B cell activation, proliferation, and differentiation. The CD52 antigen is targeted by the monoclonal antibody alemtuzumab, which is indicated for treatment of chronic lymphocytic leukemia. CD22 is targeted by a number of antibodies, and has recently demonstrated efficacy combined with toxin in chemotherapy-resistant hairy cell leukemia. Two new monoclonal antibodies targeting CD20, tositumomab and ibritumomab, have been submitted to the Food and Drug Administration (FDA). These antibodies are conjugated with radioisotopes. Alemtuzumab (Campath) is used in the treatment of chronic lymphocytic leukemia; Gemtuzumab (Mylotarg) finds use in the treatment of acute myelogenous leukemia; Ibritumomab (Zevalin) finds use in the treatment of non-Hodgkin's lymphoma; Panitumumab (Vectibix) finds use in the treatment of colon cancer.

The CD52 antigen is targeted by the monoclonal antibody alemtuzumab, which is indicated for treatment of chronic lymphocytic leukemia; colon cancer and lung cancer. CD22 is targeted by a number of antibodies, and has recently demonstrated efficacy combined with toxin in chemotherapy-resistant hairy cell leukemia.

Gemtuzumab (Mylotarg) finds use in the treatment of acute myelogenous leukemia; Ibritumomab (Zevalin) finds use in the treatment of non-Hodgkin's lymphoma; Panitumumab (Vectibix) finds use in the treatment of colon cancer.

Cetuximab (Erbitux) is also of interest for use in the methods of the invention. The antibody binds to the EGF receptor (EGFR), and has been used in the treatment of solid tumors including colon cancer and squamous cell carcinoma of the head and neck (SCCHN).

Monoclonal antibodies useful in the methods of the invention that have been used in solid tumors include, without limitation, edrecolomab and trastuzumab (herceptin). Edrecolomab targets the 17-1A antigen seen in colon and rectal cancer, and has been approved for use in Europe for these indications. Trastuzumab targets the HER-2/neu antigen. This antigen is seen on 25% to 35% of breast cancers. Cetuximab (Erbitux) is also of interest for use in the methods of the invention. The antibody binds to the EGF receptor (EGFR), and has been used in the treatment of solid tumors including colon cancer and squamous cell carcinoma of the head and neck (SCCHN).

A "patient" for the purposes of the present invention includes both humans and other animals, particularly mammals, including pet and laboratory animals, e.g. mice, rats, rabbits, *etc.* Thus the methods are applicable to both human therapy and veterinary applications. In one embodiment the patient is a mammal, preferably a primate. In other embodiments the patient is human.

The terms "subject," "individual," and "patient" are used interchangeably herein to refer to a mammal being assessed for treatment and/or being treated. In an embodiment, the mammal is a human. The terms "subject," "individual," and "patient" encompass, without limitation, individuals having cancer. Subjects may be human, but also include other mammals, particularly those mammals useful as laboratory models for human disease, e.g. mouse, rat, *etc.*

The terms "cancer," "neoplasm," and "tumor" are used interchangeably herein to refer to cells which exhibit autonomous, unregulated growth, such that they exhibit an aberrant growth phenotype characterized by a significant loss of control over cell proliferation. Cells of interest for detection, analysis, or treatment in the present application include precancerous (e.g., benign), malignant, pre-metastatic, metastatic, and non-metastatic cells. Cancers of virtually every tissue are known. The phrase "cancer burden" refers to the quantum of cancer cells or cancer volume in a subject. Reducing cancer burden accordingly refers to reducing the number of cancer cells or the cancer volume in a subject. The term "cancer cell" as used herein refers to any cell that is a cancer cell or is derived from a cancer cell e.g. clone of a cancer cell. Many types of cancers are known to those of skill in the art, including solid tumors such as carcinomas, sarcomas, glioblastomas, melanomas, lymphomas, myelomas, etc., and circulating cancers such as leukemias. Examples of cancer include but are not limited to, ovarian cancer, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, and brain cancer.

As is known in the art, cancer types can vary in the average or specific degree of mutation, where higher levels of mutation are associated with increased expression of neoantigens. See, for example, Vogelstein et al., (2013), supra. A low mutation burden can be a cancer type with an average per tumor, or specific number for an individual tumor, of up to about 10, up to about 20, up to about 30, up to about 40, up to about 50 non-synonymous mutations per tumor. A high mutation burden can be a cancer type with greater than about 50, greater than about 75, greater than about 100, greater than about 125, greater than about 150 non-synonymous mutations per tumor.

Selection of patients and tumors for CD47 blockade combination therapy with antagonistic or agonistic immunotherapies. CD47 blockade combination therapy can enhance the therapeutic response of tumors to antagonistic or agonistic immunotherapies described herein by enhancing antigen processing, presentation and T cell activation and also enhanced depletion of regulatory T cells that inhibit or eliminate anti-tumor T cells. Thus, the anti-tumor efficacy of tumors that are responsive to these therapies is enhanced and the therapeutic response of tumors that are not responsive is enabled.

A combination of CD47 blockade, with agonistic or antagonistic immune therapy described herein is given to patients with tumors subtypes that are responsive to these therapies. These tumors may be defined by a higher frequency of mutations, resulting in more tumor antigens, therefore being more immunogenic, as described above. In some embodiments patients treated with combination therapy are responsive to treatment with an immune activator or checkpoint inhibitor; however this represents a subset of approximately 25% of patients within a specific potentially responsive tumor subtype. In other embodiments, patients are treated with combination therapy of the invention that are currently-non-responsive to immunotherapies but have tumor-subtypes that are known to be responsive. This is currently a subset of approximately 75% of patients within a specific potentially responsive tumor subtype

A combination of CD47 blockade with agonistic or antagonistic therapy described herein is given to patients with tumor subtypes that are non-susceptible to these therapies. This is currently the majority of tumor subtypes. These tumors may be defined by a lower frequency of mutations, resulting in fewer tumor antigens, therefore being less immunogenic.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, *etc.*

As used herein, the terms "cancer recurrence" and "tumor recurrence," and grammatical variants thereof, refer to further growth of neoplastic or cancerous cells after diagnosis of cancer. Particularly, recurrence may occur when further cancerous cell growth occurs in the cancerous tissue. "Tumor spread," similarly, occurs when the cells of a tumor disseminate into local or distant tissues and organs; therefore tumor spread encompasses tumor metastasis. "Tumor invasion" occurs when the tumor growth spread out locally to compromise the function of involved tissues by compression, destruction, or prevention of normal organ function.

As used herein, the term "metastasis" refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part which is not directly connected to the organ of the original cancerous tumor. Metastasis can also be defined as several steps of a process, such as the departure of cancer cells from an original tumor site, and migration and/or invasion of cancer cells to other parts of the body.

The term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids, polypeptides, *etc.* The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like. A "biological sample" includes a sample obtained from a patient's cancer cell, e.g., a sample comprising polynucleotides and/or polypeptides that is obtained from a patient's cancer cell (e.g., a cell lysate or other cell extract comprising polynucleotides and/or polypeptides); and a sample comprising cancer cells from a patient. A biological sample comprising a cancer cell from a patient can also include non-cancerous cells.

The term "diagnosis" is used herein to refer to the identification of a molecular or pathological state, disease or condition, such as the identification of a molecular subtype of breast cancer, prostate cancer, or other type of cancer.

The term "prognosis" is used herein to refer to the prediction of the likelihood of cancer-attributable death or progression, including recurrence, metastatic spread, and drug resistance, of a neoplastic disease, such as ovarian cancer. The term "prediction" is used herein to refer to the act of foretelling or estimating, based on observation, experience, or scientific reasoning. In one example, a physician may predict the likelihood that a patient will survive, following surgical removal of a primary tumor and/or chemotherapy for a certain period of time without cancer recurrence.

As used herein, the terms "treatment," "treating," and the like, refer to administering an agent, or carrying out a procedure, for the purposes of obtaining an effect. The effect may be prophylactic in terms of completely or partially preventing a disease or symptom thereof and/or may be therapeutic in terms of effecting a partial or complete cure for a disease and/or symptoms of the disease. "Treatment," as used herein, may include treatment of a tumor in a mammal, particularly in a human, and includes: (a) preventing the disease or a symptom of a disease from occurring in a subject which may be predisposed to the disease but has not yet been diagnosed as having it (*e.g*., including diseases that may be associated with or caused by a primary disease; (b) inhibiting the disease, i.e., arresting its development; and (c) relieving the disease, i.e., causing regression of the disease.

Treating may refer to any indicia of success in the treatment or amelioration or prevention of an cancer, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters; including the results of an examination by a physician. Accordingly, the term "treating" includes the administration of the compounds or agents of the present invention to prevent or delay, to alleviate, or to arrest or inhibit development of the symptoms or conditions associated with cancer or other diseases. The term "therapeutic effect" refers to the reduction, elimination, or prevention of the disease, symptoms of the disease, or side effects of the disease in the subject.

"In combination with", "combination therapy" and "combination products" refer, in certain embodiments, to the concurrent administration to a patient of the agents described herein. When administered in combination, each component can be administered at the same time or sequentially in any order at different points in time. Thus, each component can be administered separately but sufficiently closely in time so as to provide the desired therapeutic effect.

"Concomitant administration" of active agents in the methods of the invention means administration with the reagents at such time that the agents will have a therapeutic effect at the same time. Such concomitant administration may involve concurrent (i.e. at the same time), prior, or subsequent administration of the agents. A person of ordinary skill in the art would have no difficulty determining the appropriate timing, sequence and dosages of administration for particular drugs and compositions of the present invention.

As used herein, endpoints for treatment will be given a meaning as known in the art and as used by the Food and Drug Administration.

Overall survival is defined as the time from randomization until death from any cause, and is measured in the intent-to-treat population. Survival is considered the most reliable cancer endpoint, and when studies can be conducted to adequately assess survival, it is usually the preferred endpoint. This endpoint is precise and easy to measure, documented by the date of death. Bias is not a factor in endpoint measurement. Survival improvement should be analyzed as a risk-benefit analysis to assess clinical benefit. Overall survival can be evaluated in randomized controlled studies. Demonstration of a statistically significant improvement in overall survival can be considered to be clinically significant if the toxicity profile is acceptable, and has often supported new drug approval. A benefit of the methods of the invention can include increased overall survival of patients.

Endpoints that are based on tumor assessments include DFS, ORR, TTP, PFS, and time-to-treatment failure (TTF). The collection and analysis of data on these time-dependent endpoints are based on indirect assessments, calculations, and estimates (e.g., tumor measurements). Disease-Free Survival (DFS) is defined as the time from randomization until recurrence of tumor or death from any cause. The most frequent use of this endpoint is in the adjuvant setting after definitive surgery or radiotherapy. DFS also can be an important endpoint when a large percentage of patients achieve complete responses with chemotherapy.

Objective Response Rate . ORR is defined as the proportion of patients with tumor size reduction of a predefined amount and for a minimum time period. Response duration usually is measured from the time of initial response until documented tumor progression. Generally, the FDA has defined ORR as the sum of partial responses plus complete responses. When defined in this manner, ORR is a direct measure of drug antitumor activity, which can be evaluated in a single-arm study.

Time to Progression and Progression-Free Survival. TTP and PFS have served as primary endpoints for drug approval. TTP is defined as the time from randomization until objective tumor progression; TTP does not include deaths. PFS is defined as the time from randomization until objective tumor progression or death. The precise definition of tumor progression is important and should be carefully detailed in the protocol.

As used herein, the term "correlates," or "correlates with," and like terms, refers to a statistical association between instances of two events, where events include numbers, data sets, and the like. For example, when the events involve numbers, a positive correlation (also referred to herein as a "direct correlation") means that as one increases, the other increases as well. A negative correlation (also referred to herein as an "inverse correlation") means that as one increases, the other decreases.

"Dosage unit" refers to physically discrete units suited as unitary dosages for the particular individual to be treated. Each unit can contain a predetermined quantity of active compound(s) calculated to produce the desired therapeutic effect(s) in association with the required pharmaceutical carrier. The specification for the dosage unit forms can be dictated by (a) the unique characteristics of the active compound(s) and the particular therapeutic effect(s) to be achieved, and (b) the limitations inherent in the art of compounding such active compound(s).

"Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients can be solid, liquid, semisolid, or, in the case of an aerosol composition, gaseous.

"Pharmaceutically acceptable salts and esters" means salts and esters that are pharmaceutically acceptable and have the desired pharmacological properties. Such salts include salts that can be formed where acidic protons present in the compounds are capable of reacting with inorganic or organic bases. Suitable inorganic salts include those formed with the alkali metals, *e.g.* sodium and potassium, magnesium, calcium, and aluminum. Suitable organic salts include those formed with organic bases such as the amine bases, *e.g*., ethanolamine, diethanolamine, triethanolamine, tromethamine, N methylglucamine, and the like. Such salts also include acid addition salts formed with inorganic acids (*e.g*., hydrochloric and hydrobromic acids) and organic acids (*e.g*., acetic acid, citric acid, maleic acid, and the alkane- and arene-sulfonic acids such as methanesulfonic acid and benzenesulfonic acid). Pharmaceutically acceptable esters include esters formed from carboxy, sulfonyloxy, and phosphonoxy groups present in the compounds, *e.g.*, C₁₋₆ alkyl esters. When there are two acidic groups present, a pharmaceutically acceptable salt or ester can be a mono-acid-mono-salt or ester or a di-salt or ester; and similarly where there are more than two acidic groups present, some or all of such groups can be salified or esterified. Compounds named in this invention can be present in unsalified or unesterified form, or in salified and/or esterified form, and the naming of such compounds is intended to include both the original (unsalified and unesterified) compound and its pharmaceutically acceptable salts and esters. Also, certain compounds named in this invention may be present in more than one stereoisomeric form, and the naming of such compounds is intended to include all single stereoisomers and all mixtures (whether racemic or otherwise) of such stereoisomers.

The terms "pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects to a degree that would prohibit administration of the composition.

A "therapeutically effective amount" means the amount that, when administered to a subject for treating a disease, is sufficient to effect treatment for that disease.

### METHODS OF USE

Methods are provided for treating or reducing primary or metastatic cancer in a regimen comprising contacting the targeted cells with a combination of (i) an agent that blockades CD47 activity; and (ii) one or more of an agent that agonizes an immune costimulatory molecule, e.g. CD40, OX40, etc.; and/or (iii) an agent that antagonizes an immune inhibitory molecule, e.g. CTLA-4, PD1, PDL1, *etc.* Such methods include administering to a subject in need of treatment a therapeutically effective amount or an effective dose of the combined agents of the invention, including without limitation combinations of the reagent with a chemotherapeutic drug, radiation therapy, or an ESA.

Immunotherapies with an agent that agonizes an immune costimulatory molecule or antagonizes an immune inhibitory molecule can induce a strong response of immune cells against tumor cells. A side effect of these therapies is toxicity to normal tissues since the activation or release of inhibition by these therapies is not antigen-specific. CD47 blockade enables a specific depletion of cancer cells by macrophages and other phagocytes/antigen immune cells. As a result, the immune response or the innate and adaptive immune system has enhanced anti-tumor specificity, with reduced toxicity to normal tissue, and therefore dose or therapy limiting side effects.

Combinations of immune regulatory agents with CD47 blockade can also enhance efficacy of the immune regulatory agents by promoting tumor antigen presentation and depletion of inhibitory immune cells. This enables a shortening of treatment period and thus reduces the duration and significance of potential toxicities and side effects.

CD47 blockade enables phagocytosis and antigen processing and presentation *in vitro.* Facilitating phagocytosis of cancer cells by phagocytes *in vitro* in the absence of normal tissue cells can prime an anti-tumor restricted immune response and therefore prevent toxicities against normal cells and tissues. Following phagocytosis of cancer cells *in vitro,* the phagocytes can be transferred into the patient to activate anti-tumor T cells *in vivo* or can be incubated *in vitro* with T cells to activate the T cells against the tumor antigens and the activated T cells can be transferred afterwards into the patient.

Effective doses of the combined agents of the present invention for the treatment of cancer vary depending upon many different factors, including means of administration, target site, physiological state of the patient, whether the patient is human or an animal, other medications administered, and whether treatment is prophylactic or therapeutic. Usually, the patient is a human, but nonhuman mammals may also be treated, e.g. companion animals such as dogs, cats, horses, *etc.,* laboratory mammals such as rabbits, mice, rats, *etc.,* and the like. Treatment dosages can be titrated to optimize safety and efficacy.

In some embodiments, the therapeutic dosage of each agent may range from about 0.0001 to 100 mg/kg, and more usually 0.01 to 5 mg/kg, of the host body weight. For example dosages can be 1 mg/kg body weight or 10 mg/kg body weight or within the range of 1-10 mg/kg. An exemplary treatment regime entails administration once every two weeks or once a month or once every 3 to 6 months. Therapeutic entities of the present invention are usually administered on multiple occasions. Intervals between single dosages can be weekly, monthly or yearly. Intervals can also be irregular as indicated by measuring blood levels of the therapeutic entity in the patient. Alternatively, therapeutic entities of the present invention can be administered as a sustained release formulation, in which case less frequent administration is required. Dosage and frequency vary depending on the half-life of the polypeptide in the patient.

In prophylactic applications, a relatively low dosage may be administered at relatively infrequent intervals over a long period of time. Some patients continue to receive treatment for the rest of their lives. In other therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

In still other embodiments, methods of the present invention include treating, reducing or preventing tumor growth, tumor metastasis or tumor invasion of cancers including carcinomas, hematologic cancers, melanomas, sarcomas, gliomas, *etc.* For prophylactic applications, pharmaceutical compositions or medicaments are administered to a patient susceptible to, or otherwise at risk of disease in an amount sufficient to eliminate or reduce the risk, lessen the severity, or delay the outset of the disease, including biochemical, histologic and/or behavioral symptoms of the disease, its complications and intermediate pathological phenotypes presenting during development of the disease.

Compositions for the treatment of cancer can be administered by parenteral, topical, intravenous, intratumoral, oral, subcutaneous, intraarterial, intracranial, intraperitoneal, intranasal or intramuscular means. A typical route of administration is intravenous or intratumoral, although other routes can be equally effective.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above. Langer, Science 249: 1527, 1990 and Hanes, Advanced Drug Delivery Reviews 28: 97-119, 1997. The agents of this invention can be administered in the form of a depot injection or implant preparation which can be formulated in such a manner as to permit a sustained or pulsatile release of the active ingredient. The pharmaceutical compositions are generally formulated as sterile, substantially isotonic and in full compliance with all Good Manufacturing Practice (GMP) regulations of the U.S. Food and Drug Administration.

Toxicity of the combined agents described herein can be determined by standard pharmaceutical procedures in cell cultures or experimental animals, e.g., by determining the LD₅₀ (the dose lethal to 50% of the population) or the LD₁₀₀ (the dose lethal to 100% of the population). The dose ratio between toxic and therapeutic effect is the therapeutic index. The data obtained from these cell culture assays and animal studies can be used in formulating a dosage range that is not toxic for use in human. The dosage of the proteins described herein lies preferably within a range of circulating concentrations that include the effective dose with little or no toxicity. The dosage can vary within this range depending upon the dosage form employed and the route of administration utilized. The exact formulation, route of administration and dosage can be chosen by the individual physician in view of the patient's condition.

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that compositions of the invention when administered orally, should be protected from digestion. This is typically accomplished either by complexing the molecules with a composition to render them resistant to acidic and enzymatic hydrolysis, or by packaging the molecules in an appropriately resistant carrier, such as a liposome or a protection barrier. Means of protecting agents from digestion are well known in the art.

The compositions for administration will commonly comprise an antibody or other ablative agent dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, e.g., sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the patient's needs (e.g., Remington's Pharmaceutical Science (15th ed., 1980) and Goodman & Gillman, The Pharmacological Basis of Therapeutics (Hardman et al., eds., 1996)).

Also within the scope of the invention are kits comprising the active agents and formulations thereof, of the invention and instructions for use. The kit can further contain a least one additional reagent, e.g. a chemotherapeutic drug, ESA, *etc.* Kits typically include a label indicating the intended use of the contents of the kit. The term label includes any writing, or recorded material supplied on or with the kit, or which otherwise accompanies the kit.

The compositions can be administered for therapeutic treatment. Compositions are administered to a patient in an amount sufficient to substantially ablate targeted cells, as described above. An amount adequate to accomplish this is defined as a "therapeutically effective dose.", which may provide for an improvement in overall survival rates. Single or multiple administrations of the compositions may be administered depending on the dosage and frequency as required and tolerated by the patient. The particular dose required for a treatment will depend upon the medical condition and history of the mammal, as well as other factors such as age, weight, gender, administration route, efficiency, etc.

### Experimental

### Example 1

### Combination of anti-CD47 antibody with agonistic antibody to CD40

Breast cancer cells (100K cells in 25% Matrigel) were injected into mice (NSG) via subcutaneous injection into the mammary fad pad and allowed to engraft. The animals were randomized into four treatment groups:
- Vehicle control (PBS)
- CD40 agonist alone (agonistic anti-CD40 antibody)
- CD47 antagonist alone (antagonistic anti-CD47 antibody)
- CD47 antagonist plus CD40 agonist

The CD47 ab (5F9) was dosed every other day, the CD40 ab (FGK) was dosed twice weekly, tumor growth was monitored by luciferase bioluminescence imaging using labeled cancer cells (e.g. luciferase positive cells).

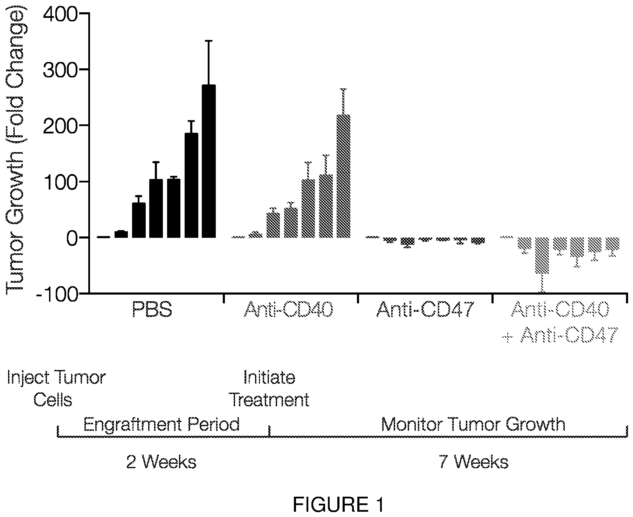

As shown in Figure 1, treatment with the anti-CD47 antibody inhibits tumor growth. Treatment with the agonistic anti-CD40 antibody produces no inhibition of tumor growth. Combination treatment with anti-CD47 antibody and anti-CD40 antibody not only produces inhibition of tumor growth but also regression of the tumor.

### Example 2

### In vitro synergy experiment

An ADCC assay is performed using mouse or human NK cells (effectors) and mouse or human cancer cells (target cells). Mouse NK cells are isolated from peripheral blood, bone marrow, or spleens; human NK cells are isolated from peripheral blood. Human cancer cell lines or primary samples are labeled for use as target cells (e.g. with chromium or fluorescent dye).

The NK cells and cancer cells are combined in vitro, and co-culture with the following treatments:
- Vehicle control (e.g. PBS)
- checkpoint inhibitor alone, including ipilimumab; nivolumab; and pembrolizumab
- CD47 antagonist alone
- CD47 antagonist plus checkpoint inhibitor

ADCC is measured via chromium-release assay or flow cytometry cell death assays (e.q. Annexin V/DAPI staining). NK cell cytokine (e.g. IFN-gamma) release is measured via ELISA. The change in cell death and cytokine release in the presence of checkpoint inhibitor combined with anti-CD47 is determined relative to the mono-therapies listed above.

### Example 3

### In vivo experiment protocol

Cancer cells are injected into mice via subcutaneous, retroperitoneal, or peripheral blood injection and allowed to engraft. The animals are randomized into four treatment groups:
- Vehicle control (e.g. PBS)
- checkpoint inhibitor alone, including ipilimumab; nivolumab; and pembrolizumab
- CD47 antagonist alone
- CD47 antagonist plus checkpoint inhibitor

Mice are treated daily, three times per week, twice per week, or once per week with the respective treatments. Tumor burden is measured by tumor volume measurements, bioluminescence using labeled cancer cells (e.g. luciferase positive cells), and/or analysis of peripheral blood. The overall survival of the mice is also measured.

### Example 4

### T cell Assays

Antigen presentation assay. For *in vitro* antigen presentation assays, 10⁴ macrophages are cocultured with equal numbers of DLD1-cOVA-GFP cancer cells overnight in serum-free RPMI media. The following day, equal volume of RPMI + 20% FCS is added to the cultures. Peripheral lymph nodes are harvested from OT-I or OT-II TCR transgenic mice and labeled with 0.5 mM CFSE (Molecular Probes). T cells are isolated using biotinylated anti-CD8 or anti-CD4 antibodies, followed by enrichment with anti-biotin magnetic beads (Miltenyi Biotec). 5 x 10⁴ T cells are added to the cultures and analyzed at day 3 (for OT-I T cells) or day 4 (for OT-II T cells). For in vivo antigen presentation assays, 2 x 10⁶ CFSE-labeled OT-I T cells (CD45.2) are adoptively transferred iv into recipient mice (CD45.1). Macrophages are isolated from co-culture with cancer cells and injected into the footpad of mice. Popliteal lymph nodes are analyzed on day 4 for CFSE dilution within CD45.2+ cells.

In vivo cell killing assay. In brief, splenocytes from C57BL/Ka (CD45.1) mice are labeled with 10 µM CFSE (CFSE-high) and 1 µM CFSE (CFSE-low). CFSE-high splenocytes are pulsed in a 6-well plate with 1 µM SIINFEKL peptide for 1 hour. Cells are mixed in a 1:1 ratio with non-peptide-pulsed CFSE-low cells before iv transfer. To account for variation in the CFSE high/low ratio in the absence of peptide-specific lysis, control mice receive CFSE-high splenocytes not pulsed with SIINFEKL peptide before mixing in a 1:1 ratio with CFSE-low splenocytes and transfer to mice. Draining lymph nodes are analyzed 16 hours later. Percent cytotoxicity was calculated as (1 - %CFSE^{high}/%CFSE^{low}) normalized to the ratio in control mice receiving splenocytes not pulsed with SIINFEKL peptide.

Tumor challenge. 1 × 10⁶ CD8-enriched OT-I T cells are adoptively transferred iv into recipient C57BL/Ka mice. Macrophages from syngeneic C57BL/Ka mice are co-cultured with DLD1-cOVA-GFP cancer, and then isolated by magnetic enrichment and injected into the footpad of mice. The tumor cell line E.G7 (EL.4 cells expressing the chicken OVA cDNA) is used for tumor challenge of mice (ATCC). 1 × 105 E.G7 cells are injected s.c. into the right hindlimb of the mice in a 1:1 ratio with regular matrigel. Tumor size is measured every day by using fine calipers and volume calculated based on length * width * height * π/6.

T Cell Proliferation. Mature T cells recognize and respond to the antigen/MHC complex through their antigen-specific receptors (TCR). The most immediate consequence of TCR activation is the initiation of signaling pathways including induction of specific protein tyrosine kinases (PTKs), breakdown of phosphatidylinositol 4,5-biphosphate (PIP2), activation of protein kinase C (PKC) and elevation of intracellular calcium ion concentration. These early events are transmitted to the nucleus and result in clonal expansion of T cells; upregulation of activation markers on the cell surface; differentiation into effector cells; induction of cytotoxicity or cytokine secretion; induction of apoptosis.

T cell activation is assessed by measuring T cell proliferation upon in vitro stimulation of T cells via antigen or agonistic antibodies to TCR. This protocol is written as a starting point for examining in vitro proliferation of mouse splenic T-cells and human peripheral T cells stimulated via CD3. Critical parameters include cell density, antibody titer and activation kinetics.

Prepare a 5-10 µg/mL solution of anti-CD3e (145-2C11) in sterile PBS. Calculate the number of wells required for each experimental condition and consider triplicate samples for each condition. For example, to coat one-half plate (48 wells) 2.6mL of antibody solution is required. Dispense 50 µL of the antibody solution to each well of the 96-well assay plate. For the control unstimulated wells, add 50 µL of sterile PBS. Tightly cover the plate with ParafilmTM to avoid sample evaporation and incubate at 37°C for 2 hours or prepare the plate one day in advance and keep at 4°C overnight. Just before adding cells, remove the 50 µL antibody solution with a multichannel pipettor. Rinse each well with 200 µL of sterile PBS and discard PBS.

Harvest spleen and prepare a single cell suspension under sterile conditions and resuspend in complete RPMI-1640 at 10⁶/mL in the presence of the desired agents, e.g. anti-CD47, checkpoint inhibitors, etc. Add 200 µL of the cell suspension to each well and place in a humidified 37°C, 5% CO2 incubator. Add soluble anti-CD28 to cells at 2 µg/mL. Incubate for 2-4 days. Cells can be harvested and processed for quantitation.

### Example 5

### A Study Of Avelumab In Combination With CD47 Blockade In Advanced Malignancies (JAVELIN Medley)

This is dose-optimization study to evaluate safety, pharmacokinetics, pharmacodynamics, and antitumor activity of avelumab (MSB0010718C) in combination with CD47 blockade and other cancer immunotherapies in patients with locally advanced or metastatic solid tumors [eg, non-small cell lung cancer (NSCLC), melanoma, and squamous cell carcinoma of the head and neck (SCCHN)]. The primary purpose is to assess the safety and efficacy of various combinations with CD47 blockade, optimizing dosing regimens as appropriate, in a limited series of indications. Initially, the study will evaluate the safety and antitumor activity of avelumab, an anti-PD-L1 monoclonal antibody (mAb) in combination with 5F9-G4, a humanized antibody that blocks interactions between CD47 and SIRPα.

Primary Outcome Measures: Number of participants with Dose-Limiting Toxicities (DLT) occurring during the first 8 weeks of treatment (first 2 cycles).

Objective Response - Number of Participants With Objective Response ie, confirmed complete or partial response according to RECIST Version 1.1). Time to Tumor Response (TTR) is defined for patients with confirmed objective response (CR or PR) as the time from the date of randomization (NSCLC) or date of first dose of study treatment (melanoma and SCCHN) to the first documentation of objective tumor response. Duration of Response (DR) is defined for patients with confirmed objective response (CR or PR) as the time from the first documentation of objective tumor response to the first documentation of objective tumor progression or to death due to any cause, whichever occurs first. Progression-Free Survival (PFS) is defined as the time from the date of randomization (NSCLC) or date of first dose of study treatment (melanoma and SCCHN) to the date of disease progression by RECIST v1.1 or death due to any cause, whichever occurs first. Overall Survival (OS) is defined as the time from the date of randomization (NSCLC) or date of first dose of study treatment (melanoma and SCCHN) to the date of death.

| Arms | Assigned Interventions |
|---|---|
| Experimental: Cohort A1 | Biological: Avelumab |
| NSCLC patients treated with 10mg/kg avelumab + 5F9-G4 Ab | Anti-PD-L1 antibody at 10 mg/kg IV every 2 weeks until disease progression. |
| | Biological: 5F9-G4 Ab |
| | following a priming dose of 1mg/kg, Hu5F9-G4 will be dosed at increasing dose levels ranging from 1-100mg/kg. Antibody will be administered to optimize the combination with avelumab. Treatment with the combination will continue until disease progression. |
| Experimental: Cohort A2 | Biological: Avelumab |
| Melanoma patients treated with 10mg/kg avelumab + 5F9-G4 Ab | Anti-PD-L1 antibody at 10 mg/kg IV every 2 weeks until disease progression. |
| | Biological: 5F9-G4 Ab |
| | following a priming dose of 1mg/kg, Hu5F9-G4 will be dosed at increasing dose levels ranging from 1-100mg/kg. Antibody will be administered to optimize the combination with avelumab. Treatment with the combination will continue until disease progression. |
| Experimental: Cohort A3 | Biological: Avelumab |

| | |
|---|---|
| SCCHN patients treated with 10mg/kg avelumab + 5F9-G4 Ab | Anti-PD-L1 antibody at 10 mg/kg IV every 2 weeks until disease progression. |
| | Biological: 5F9-G4 Ab |
| | following a priming dose of 1mg/kg, Hu5F9-G4 will be dosed at increasing dose levels ranging from 1-100mg/kg. Antibody will be administered to optimize the combination with avelumab. Treatment with the combination will continue until disease progression. |

### Example 7

### Anti-OX40 Antibody in Combination with CD47 Blockade for Head and Neck Cancer Patients

This is dose-optimization study to evaluate safety, pharmacokinetics, pharmacodynamics, and antitumor activity of the anti-OX40 antibody, MEDI6469 in combination Primary Outcome Measures:

Primary Outcome Measures: Number of participants with Dose-Limiting Toxicities (DLT) occurring during the first 8 weeks of treatment (first 2 cycles).

Objective Response - Number of Participants With Objective Response ie, confirmed complete or partial response according to RECIST Version 1.1). Time to Tumor Response (TTR) is defined for patients with confirmed objective response (CR or PR) as the time from the date of randomization (NSCLC) or date of first dose of study treatment (melanoma and SCCHN) to the first documentation of objective tumor response. Duration of Response (DR) is defined for patients with confirmed objective response (CR or PR) as the time from the first documentation of objective tumor response to the first documentation of objective tumor progression or to death due to any cause, whichever occurs first. Progression-Free Survival (PFS) is defined as the time from the date of randomization (NSCLC) or date of first dose of study treatment (melanoma and SCCHN) to the date of disease progression by RECIST v1.1 or death due to any cause, whichever occurs first. Overall Survival (OS) is defined as the time from the date of randomization (NSCLC) or date of first dose of study treatment (melanoma and SCCHN) to the date of death.

| Arms | Assigned Interventions |
|---|---|
| Anti-OX40 antibody administration + 5F9-G4 Ab | Drug: Anti-OX40 antibody administration |
| | Anti-OX40 antibody administration at 0.4 mg/kg IV x 3 doses given on Days 1, 3 or 4, and 5 or 6 of study |
| | Biological: 5F9-G4 Ab |
| | following a priming dose of 1mg/kg, Hu5F9-G4 will be dosed at increasing dose levels ranging from 1-100mg/kg. Antibody will be administered to optimize the combination with avelumab. Treatment with the combination will continue until disease progression. |

### Example 8

### A Study Of 4-1BB Agonist PF-05082566 Plus 5F9-G4 anti-CD47 Antibody In Patients With Solid Tumors

Primary Outcome Measures: Number of participants with Dose-Limiting Toxicities (DLT) occurring during the first 8 weeks of treatment (first 2 cycles).

Objective Response - Number of Participants With Objective Response ie, confirmed complete or partial response according to RECIST Version 1.1). Time to Tumor Response (TTR) is defined for patients with confirmed objective response (CR or PR) as the time from the date of randomization (NSCLC) or date of first dose of study treatment (melanoma and SCCHN) to the first documentation of objective tumor response. Duration of Response (DR) is defined for patients with confirmed objective response (CR or PR) as the time from the first documentation of objective tumor response to the first documentation of objective tumor progression or to death due to any cause, whichever occurs first. Progression-Free Survival (PFS) is defined as the time from the date of randomization (NSCLC) or date of first dose of study treatment (melanoma and SCCHN) to the date of disease progression by RECIST v1.1 or death due to any cause, whichever occurs first. Overall Survival (OS) is defined as the time from the date of randomization (NSCLC) or date of first dose of study treatment (melanoma and SCCHN) to the date of death.

| Arms | Assiqned Interventions |
|---|---|
| Experimental: PF-05082566 +5F9-G4 | Drug: PF-05082566 |
| | Starting dose of 0.45 mg/kg q3wks IV, dose escalation |
| | Drug: 5F9-G4 |
| | following a priming dose of 1mg/kg, Hu5F9-G4 will be dosed at increasing dose levels ranging from 1-100mg/kg, IV |

### Example 9

### A Study Of PF-06801591 in combination with 5F9-G4 In Melanoma, Head And Neck Cancer (SCHNC), Ovarian, Sarcoma, Hodgkin Lymphoma

Primary Outcome Measures: Number of participants with Dose-Limiting Toxicities (DLT) occurring during the first 8 weeks of treatment (first 2 cycles).

Objective Response - Number of Participants With Objective Response ie, confirmed complete or partial response according to RECIST Version 1.1). Time to Tumor Response (TTR) is defined for patients with confirmed objective response (CR or PR) as the time from the date of randomization (NSCLC) or date of first dose of study treatment (melanoma and SCCHN) to the first documentation of objective tumor response. Duration of Response (DR) is defined for patients with confirmed objective response (CR or PR) as the time from the first documentation of objective tumor response to the first documentation of objective tumor progression or to death due to any cause, whichever occurs first. Progression-Free Survival (PFS) is defined as the time from the date of randomization (NSCLC) or date of first dose of study treatment (melanoma and SCCHN) to the date of disease progression by RECIST v1.1 or death due to any cause, whichever occurs first. Overall Survival (OS) is defined as the time from the date of randomization (NSCLC) or date of first dose of study treatment (melanoma and SCCHN) to the date of death.

| Arms | Assigned Interventions |
|---|---|
| Experimental: Arm 1 PF-06801591 + 5F9-G4 Ab PF-06801591 0.5 mg/kg every 21 days | Drug: PF-06801591 |
| | IV every 21 days |
| | Drug: 5F9-G4 |
| | following a priming dose of 1mg/kg, Hu5F9-G4 will be dosed at increasing dose levels ranging from 1-100mg/kg IV |
| Experimental: Arm 2 PF-06801591 + 5F9-G4 Ab PF-06801591 1.0 mg/kg every 21 days | Drug: PF-06801591 |
| | IV every 21 days |
| | following a priming dose of 1mg/kg, Hu5F9-G4 will be dosed at increasing dose levels ranging from 1-100mg/kg IV |
| Experimental: Arm 3 PF-06801591+ 5F9-G4 Ab PF-06801591 3.0 mg/kg every 21 days | Drug: PF-06801591 |
| | IV every 21 days |
| | Drug: 5F9-G4 |
| | following a priming dose of 1mg/kg, Hu5F9-G4 will be dosed at increasing dose levels ranging from 1-100mg/kg, IV |
| Experimental: Arm 4 PF-06801591 + 5F9-G4 Ab | Drug: PF-06801591 |
| | IV every 21 days |
| PF-06801591 10 mg/kg every 21 days | Drug: 5F9-G4 |
| | following a priming dose of 1mg/kg, Hu5F9-G4 will be dosed at increasing dose levels ranging from 1-100mg/kg, IV |

### Example 10

### A Study of 5F9-G4 In Combination With Mogamulizumab In Patients With Advanced Solid Tumors or Lymphoma (including T cell Lymphoma)

Primary Outcome Measures: Number of participants with Dose-Limiting Toxicities (DLT) occurring during the first 8 weeks of treatment (first 2 cycles).

Objective Response - Number of Participants With Objective Response ie, confirmed complete or partial response according to RECIST Version 1.1, Immune-Related Response Criteria, the International Working Group (IWG) or Customized Response Criteria for Lymphoma. Time to Tumor Response (TTR) is defined for patients with confirmed objective response (CR or PR) as the time from the date of randomization (NSCLC) or date of first dose of study treatment (melanoma and SCCHN) to the first documentation of objective tumor response. Duration of Response (DR) is defined for patients with confirmed objective response (CR or PR) as the time from the first documentation of objective tumor response to the first documentation of objective tumor progression or to death due to any cause, whichever occurs first. Progression-Free Survival (PFS) is defined as the time from the date of randomization (NSCLC) or date of first dose of study treatment (melanoma and SCCHN) to the date of disease progression by RECIST v1.1 or death due to any cause, whichever occurs first. Overall Survival (OS) is defined as the time from the date of randomization (NSCLC) or date of first dose of study treatment (melanoma and SCCHN) to the date of death.

| Arms | Assigned Interventions |
|---|---|
| Experimental: 5F9-G4 + KW-0761 | Drug: 5F9-G4 |
| During Parts 1 & 2 Mogamulizumab and 5F9-G4 will be administered at appropriate intervals. Part 1: 5F9-G4 1 dose escalation; increased doses of 5F9-G4 IV are administered with mogamulizumab IV. Part 2: patients will be treated with the maximum tolerated dose established in Phase 1 for the combination. | Part 1: following a priming dose of mg/kg, Hu5F9-G4 will be dosed at increasing dose levels ranging from 1-100mg/kg |
| | Part 2: MTD of 5F9-G4 IV established in Part 1 is administered. |
| | Drug: KW-0761 |
| | Part 1: KW-0761 IV administered at appropriate intervals. Part 2: KW-0761 IV administered at appropriate intervals at the MTD dose for the combination. Other Name: KW-0761 = Mogamulizumab or POTELIGEO^{®} |

### Example 11

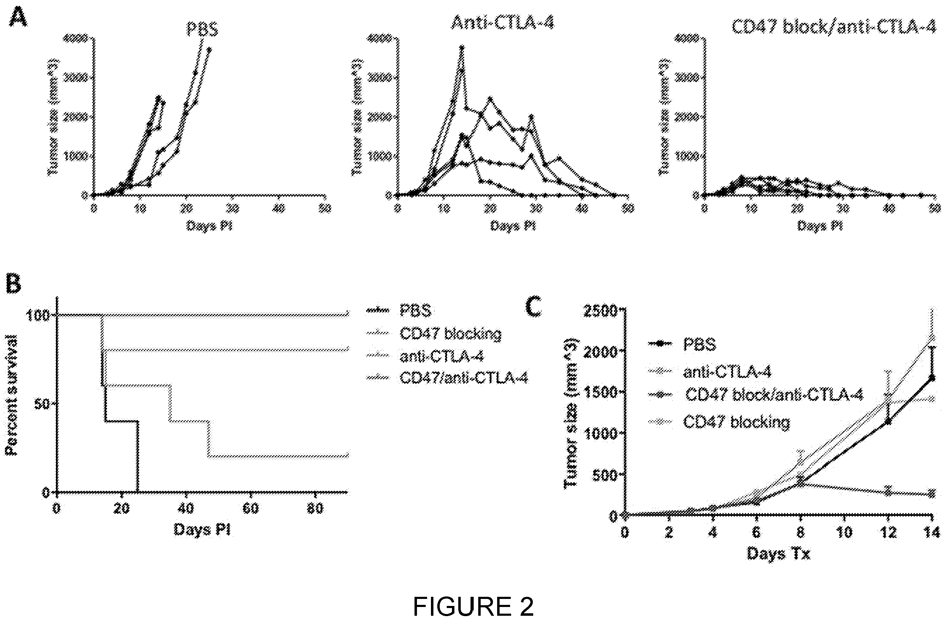

CD47 blockade synergizes with current immunotherapies including anti-CTLA-4. Wild-type 129 male mice were implanted with 1 × 10⁶ MCA sarcoma cells subcutaneously. Mice were treated IP with a CD47 blocking agent, anti-CTLA-4, or a combination. Tumor size was measured by caliper measurements and animals were euthanized when tumors exceed 2cm in any direction. Data is shown in Figure 2. A) Spider plots from N=5 mice for PBS, anti-CTLA-4, or CD47 blocking/anti-CTLA-4 combination. B) Survival plot of mice receiving individual or combination therapy. C) Tumor size (mm³)of single and combination therapy.

Each publication cited in this specification is hereby incorporated by reference in its entirety for all purposes.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention, which will be limited only by the appended claims

As used herein the singular forms "a", "and", and "the" include plural referents unless the context clearly dictates otherwise. Thus, for example, reference to "a cell" includes a plurality of such cells and reference to "the culture" includes reference to one or more cultures and equivalents thereof known to those skilled in the art, and so forth. All technical and scientific terms used herein have the same meaning as commonly understood to one of ordinary skill in the art to which this invention belongs unless clearly indicated otherwise.

Each publication cited in this specification is hereby incorporated by reference in its entirety for all purposes.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention, which will be limited only by the appended claims

As used herein the singular forms "a", "and", and "the" include plural referents unless the context clearly dictates otherwise. Thus, for example, reference to "a cell" includes a plurality of such cells and reference to "the culture" includes reference to one or more cultures and equivalents thereof known to those skilled in the art, and so forth. All technical and scientific terms used herein have the same meaning as commonly understood to one of ordinary skill in the art to which this invention belongs unless clearly indicated otherwise.

### Further embodiments of the invention:

1. A method of targeting cancer cells for immunodepletion, the method comprising:
   contacting a population of cells comprising the targeted cells with a combination of (i) an agent that blockades CD47 activity; and (ii) one or more of an agent that agonizes an immune costimulatory molecule and/or (iii) an agent that antagonizes an immune inhibitory molecule, in a dose effective to increase depletion of the targeted cells.
2. The method of embodiment 1, wherein the combination comprises an antibody that agonizes an immune costimulatory molecule.
3. The method of embodiment 2, wherein the antibody is an agonist of CD40.
4. The method of embodiment 2 wherein the antibody is an agonist of OX40.
5. The method of embodiment 1, wherein the combination comprises an antibody that antagonizes an immune inhibitory molecule.
6. The method of embodiment 5, wherein the antibody is an antagonist of CTLA4.
7. The method of embodiment 5, wherein the antibody is an antagonist of PD1 and/or PDL1.
8. The method of embodiment 5, wherein the antibody is an antagonist of TIM3.
9. The method of embodiment 5, wherein the antibody is an antagonist of LAG3.
10. The method of any one of embodiments 1-9, further comprising an antibody that binds to an antigen on the targeted tumor cell.
11. The method of any of embodiments 1-10, wherein the contacting is performed in vitro.
12. The method of any of embodiments 1-10, wherein the contacting is performed on an individual mammal in vivo.
13. The method of embodiment 12, wherein the treatment provides for increased overall survival of the individual.
14. The method of embodiment 13, wherein depletion of the target cells is enhanced relative to the depletion observed with a monotherapy of any single agent administered as a monotherapy.
15. The method of any of embodiments 1-14, wherein the agent that agent that blockades CD47 activity is an anti-CD47 antibody.
16. The method of embodiment 15, wherein the anti-CD47 antibody comprises an IgG4 Fc region.
17. The method of embodiment 16 wherein the antibody is 5F9-G4.
18. The method according to any of embodiments 1-17, wherein said mammal is a mouse.
19. The method according to any of embodiments 1-17, wherein said mammal is a human.
20. The method of embodiment 12, further comprising administration of a priming dose of the agent that blockades CD47 activity.
21. The method of embodiment 12, further comprising administering a priming dose of an erythropoietin stimulating agent.
22. The method of any of embodiments 1-21, wherein the combination of an agent that (i) an agent that blockades CD47 activity; and (ii) one or more of an agent that agonizes an immune costimulatory molecule and/or (iii) an agent that antagonizes an immune inhibitory molecule, in a dose effective to increase depletion of the targeted cells, provides for a therapeutically effective dose of agents with a clinically significant reduction in immune-related adverse events relative to the dosing required for efficacy in the absence of the anti-CD47 agent.

## Claims

1. An anti-CD47 antibody, for use in a method of targeting cancer cells for immunodepletion, the method comprising:
contacting a population of cells comprising the targeted cells with a combination of (i) the anti-CD47 antibody; and (ii) an antibody that is an antagonist of CTLA4, in a dose effective to increase depletion of the targeted cells, wherein the contacting is performed on an individual mammal in vivo.

2. An antibody that is an antagonist of CTLA4, for use in a method of targeting cancer cells for immunodepletion, the method comprising:
contacting a population of cells comprising the targeted cells with a combination of (i) an anti-CD47 antibody; and (ii) the antibody that is an antagonist of CTLA4, in a dose effective to increase depletion of the targeted cells, wherein the contacting is performed on an individual mammal in vivo.

3. An anti-CD47 antibody and an antibody that is an antagonist of CTLA4, for use in a method of targeting cancer cells for immunodepletion, the method comprising:
contacting a population of cells comprising the targeted cells with a combination of (i) the anti-CD47 antibody; and (ii) the antibody that is an antagonist of CTLA4, in a dose effective to increase depletion of the targeted cells, wherein the contacting is performed on an individual mammal in vivo.

4. The antibody or antibodies for use of any one of claims 1-3, further comprising an antibody that binds to an antigen on the targeted tumor cell.

5. The antibody or antibodies for use of any preceding claim, wherein:
(a) the treatment provides for increased overall survival of the individual, and/or
(b) the route of administration is intravenous or intratumoral.

6. The antibody or antibodies for use of any preceding claim, wherein:
a. the method further comprises administration of a priming dose of the agent that blockades CD47 activity; or
b. the method further comprises administering a priming dose of an erythropoietin stimulating agent.

7. The antibody or antibodies for use of any preceding claim, wherein the combination of antibodies provides a synergistic effect relative to the administration of the anti-CD47 antibody or the antibody that is an antagonist of CTLA4 as a monotherapy.

8. The antibody or antibodies for use of any preceding claim, wherein the combination of antibodies is administered within a period of time to produce a synergistic effect on depletion of cancer cells in the host.

9. The antibody or antibodies for use of any preceding claim, wherein the anti-CD47 antibody synergizes with the antibody that is an antagonist of CTLA4 and enhances the potency of the antibody that is an antagonist of CTLA4 to stimulate ADCP and ADCC.

10. The antibody or antibodies for use of any preceding claim, wherein the anti-CD47 antibody comprises an IgG4 Fc region, optionally wherein the antibody is 5F9-G4.

11. The antibody or antibodies for use of any preceding claim, wherein the antibody that is an antagonist of CTLA4 comprises ipilimumab or tremelimumab.

12. The antibody or antibodies for use of any preceding claim, wherein said mammal is a human.

13. The antibody or antibodies for use of any preceding claim, wherein said mammal is a mouse.

14. The antibody or antibodies for use of any preceding claim, wherein the cancer cells are:
(a) solid tumor cells, optionally wherein the cancer cells are carcinoma, sarcoma, glioblastoma, melanoma, lymphoma, or myeloma cells,
(b) circulating cancer cells, optionally wherein the cancer cells are leukemia cells, or
(c) ovarian cancer, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, or brain cancer cell.

15. The antibody or antibodies for use of claim 14, wherein the cancer cells are sarcoma cells.
